# EUROPÄISCHE PATENTSCHRIFT

(11) **EP 3 750 869 B1**
(45) Veröffentlichungstag und Bekanntmachung des Hinweises auf die Patenterteilung: **10.08.2022**
(21) Anmeldenummer: 20178641.5
(22) Anmeldetag: 08.06.2020
(51) Int. Cl.: C07C 209/00, C07C 209/90, C07C 263/10, C07C 211/12, C07C 211/09, C07C 211/51, C07C 265/14

(54) **VERFAHREN ZUR HERSTELLUNG EINER AMIN-HYDROCHLORID-SUSPENSION**
METHOD FOR PRODUCING AN AMINE-HYDROCHLORIDE SUSPENSION
PROCÉDÉ DE PRODUCTION D'UNE SUSPENSION D'HYDROCHLORURE D'AMINE

(30) Priorität: 11.06.2019 EP 19179482
(43) Veröffentlichungstag der Anmeldung: 16.12.2020
(73) Patentinhaber: Covestro Intellectual Property GmbH & Co. KG, 51373 Leverkusen (DE)
(72) Erfinder: Merkel, Michael, 40223 Düsseldorf (DE); Loddenkemper, Tim, 41542 Dormagen (DE); Gerhartz, Frank, 51381 Leverkusen (DE); Hirschfeld, Stefan, 46519 Alpen (DE)
(74) Vertreter: Levpat

(56) Entgegenhaltungen:
- EP-A1- 0 384 463
- US-A- 5 136 086

## Beschreibung

Die vorliegende Erfindung betrifft ein Verfahren zur Herstellung einer Suspension eines Hydrochlorids eines organischen Amins, umfassend die folgenden Schritte (i) Vorlegen mindestens eines organischen Lösungsmittels in einem Reaktionsbehälter unter Bildung eines Flüssigkeitsspiegels, (ii) Zugeben von Chlorwasserstoff, (iii) Zugeben des organischen Amins, wobei die Zugabe des organischen Amins unterhalb des im Reaktionsbehälters vorhandenen Flüssigkeitsspiegels erfolgt und die Schritte (ii) und (iii) zumindest teilweise gleichzeitig durchgeführt werden. Des Weiteren betrifft die vorliegende Erfindung auch ein Verfahren, wobei die nach Schritt (iii) erhaltene Suspension in einem Schritt (iv) mit Phosgen unter Erhalt des dem eingesetzten organischen Amin entsprechenden organischen Isocyanates umgesetzt wird, das entsprechende organische Isocyanat und die Verwendung dieses organischen Isocyanates zur Herstellung von Polyisocyanaten.

Es ist bekannt, dass organische Isocyanate durch Umsetzung von Phosgen mit den entsprechenden Aminen hergestellt werden können. Insbesondere bei der Umsetzung aliphatischer Amine zu den entsprechenden aliphatischen Isocyanaten entstehen aufgrund der hohen Reaktivität stets hohe Gehalte an unerwünschten Nebenprodukten, beispielsweise harzartige, schwersiedende Verbindungen oder chlorierte Verbindungen, die durch Desaminierung entstehen.

Es hat deshalb nicht an Versuchen gemangelt, diese Probleme zu vermeiden oder zumindest zu verringern. So beschreibt beispielsweise die DE 1 593 588 die Phosgenierung von Xylylendiamin, wobei in der Heißphosgenierung mit einem Phosgenüberschuss bei 120 bis 180 °C und einem Druck von 2 bis 5 atü gearbeitet wird. Auch mit diesem Verfahren entstanden höchstens ca. 90% des gewünschten Xylylendiisocyanat und entsprechend 10% und mehr an unerwünschten Nebenprodukten.

In der EP 1 908 749 wird eine Variante des Verfahrens beschrieben, bei dem das Amin in der so genannten Basenphosgenierung nicht direkt mit Phosgen umgesetzt wird, sondern das Amin wird zunächst mit Chlorwasserstoff zum entsprechenden Aminhydrochlorid umgesetzt und dieses wird dann phosgeniert. Es wird darauf hingewiesen, dass mit den Verfahren gemäß Stand der Technik, insbesondere bei Amin-Konzentrationen oberhalb von 5 Gew.-% im Lösungsmittel, sehr hochviskose Suspensionen erhalten werden, die eine schlechte Fließ- und Pumpfähigkeit und damit auch eine schlechte Rührfähigkeit aufweisen. Das Problem wurde gemäß der genannten Schrift gelöst, indem die Bildung des Hydrochlorids unter einem Druck betrieben wird, der mindestens 0,01 MPa über dem Atmosphärendruck liegt.

Nachteilig an einem solchen Verfahren ist der Betrieb unter Überdruck, der erhöhte Anforderungen an die Apparate stellt und im Falle einer Außenleckage das Gefahrenpotenzial durch austretende, gasförmige Gefahrstoffe erhöht.

Auch die DE 69 011 358 (entspricht der europäischen Anmeldung EP0384463) beschreibt ein Verfahren zur Herstellung von Xylylendiisocyanat, bei dem Xylylendiaminhydrochlorid mit Phosgen in Gegenwart eines Esters als Reaktionslösungsmittel umgesetzt wird. Das Xylylendiamin wird dabei in einer Ausführungsform im ersten Schritt bei einer Temperatur von 30 °C oder weniger mit Chlorwasserstoff in das Hydrochlorid überführt und später bei 120 bis 170 °C phosgeniert. Aufgrund der exothermen Reaktion ist für diese Vorgehensweise eine starke Kühlung notwendig, um die Bildung von Carbonsäureamiden durch Aminolyse des Lösungsmittels zu minimieren. Gänzlich verhindern lässt sie sich allerdings nicht. Auf der anderen Seite wird empfohlen, die Temperatur nicht unter 0 °C abzusenken, so dass letztlich nur ein enges Prozessfenster verbleibt. Des Weiteren ist man gemäß diesem Verfahren auf die Verwendung von Estern als Reaktionslösungsmittel beschränkt, wobei als Nebenreaktion die bereits genannte Aminolyse des Lösungsmittels auftritt. Üblicherweise in der Phosgenierung bevorzugte chlorierte aromatische Lösungsmittel wie Chlorbenzol oder ortho-Dichlorbenzol, welche sich aufgrund ihrer physikalischen und chemischen Eigenschaften in besonderer Weise für die Herstellung von Isocyanaten durch Phosgenierung der entsprechenden Amine eignen, können in diesem Verfahren nicht verwendet werden.

US5136086 offenbart die Herstellung aliphatischer Isocyanate ausgehend von entsprechenden Diaminen (wie z.B. Hexamethylendiamin) mit Phosgen in Gegenwart eines Esters, wobei vor der Phosgenierung das Amin, wie in der zuvor genannten DE69011358, zuerst ebenfalls in das Hydrochlorid überführt wird.

Ausgehend von diesem Stand der Technik lag der vorliegenden Erfindung die Aufgabe zu Grunde, die aus dem Stand der Technik bekannten Nachteile zu vermeiden, insbesondere war es Aufgabe der vorliegenden Erfindung, auch bei Normaldruck bzw. in drucklosen Verfahren und bei Temperaturen oberhalb von 30 °C eine rühr- und pumpfähige Suspension enthaltend das jeweilige Hydrochlorid von organischen Aminen zu erhalten. Eine weitere Aufgabe der Erfindung war die wirtschaftliche, effiziente und einfache Herstellung aliphatischer Isocyanate, bevorzugt aliphatischer Diisocyanate, durch Phosgenierung von entsprechend hergestellten Aminhydrochloriden, die in Suspension vorliegen.

Diese Aufgabe wurde erfindungsgemäß gelöst durch das Verfahren zur Herstellung einer Suspension eines Hydrochlorids eines organischen Amins, umfassend die folgenden Schritte:
(i) Vorlegen mindestens eines organischen Lösungsmittels in einem Reaktionsbehälter unter Bildung eines Flüssigkeitsspiegels,
(ii) Zugeben des organischen Amins,
(iii) Zugeben von Chlorwasserstoff, wobei die Zugabe des organischen Amins unterhalb des im Reaktionsbehälter vorhandenen Flüssigkeitsspiegels erfolgt und die Schritte (ii) und (iii) zumindest teilweise gleichzeitig durchgeführt werden.

Die einzelnen Schritte des erfindungsgemäßen Verfahrens werden im Folgenden detailliert beschrieben.

Schritt (i) des erfindungsgemäßen Verfahrens umfasst das Vorlegen mindestens eines organischen Lösungsmittels in einem Reaktionsbehälter unter Bildung eines Flüssigkeitsspiegels.

Im Rahmen der vorliegenden Erfindung können im Allgemeinen alle dem Fachmann für die Herstellung von Hydrochloriden von organischen Aminen als geeignet erscheinenden organischen Lösungsmittel verwendet werden.

Erfindungsgemäß bevorzugt wird ein aprotisches organisches Lösungsmittel eingesetzt. Besonders bevorzugt ist das mindestens eine organische Lösungsmittel ausgewählt aus der Gruppe bestehend aus aromatischen Kohlenwasserstoffen, halogenierten aromatischen Kohlenwasserstoffen, insbesondere chlorierten aromatischen Kohlenwasserstoffen, Estern, Ethern, halogenierten Kohlenwasserstoffen und Mischungen davon.

Ein erfindungsgemäß besonders bevorzugt eingesetzter aromatischer Kohlenwasserstoff ist Toluol.

Erfindungsgemäß besonders bevorzugt eingesetzte halogenierte Kohlenwasserstoffe sind ausgewählt aus der Gruppe bestehend aus Brombenzol, Chlorbenzol, Dichlorbenzol, beispielsweise ortho-Dichlorbenzol, und Mischungen davon.

Ganz besonders bevorzugt werden erfindungsgemäß Chlorbenzol, Dichlorbenzol, beispielsweise ortho-Dichlorbenzol, oder Mischungen davon als organisches Lösungsmittel eingesetzt.

Das erfindungsgemäße Verfahren kann im Allgemeinen in jedem dem Fachmann als geeignet erscheinenden Reaktor durchgeführt werden. Durch die Schritte (ii) und (iii) kann eine ausreichende Durchmischung stattfinden. Für den Fachmann ist ersichtlich, dass die Verwendung einer darüber hinaus gehenden Mischvorrichtung nicht zwingend erforderlich ist, aber eine vorteilhafte Ausgestaltung ist.

Als Reaktor eignet sich beispielsweise ein Rührbehälter. Ein geeigneter Rührbehälter ist beispielsweise in der DE 19957816 A1 beschrieben. Vorzugsweise handelt es sich um einen rotationssymmetrischen Rührbehälter mit vertikal stehender Hauptachse. Der Rührbehälter kann entlang dieser Hauptachse unterschiedliche Durchmesser aufweisen, ist jedoch bevorzugt im Wesentlichen zylindrisch. Boden und Deckel können beispielsweise als Klöpperboden oder auch flach ausgeführt sein. Zur Temperierung kann der Rührbehälter mit Wärmetauscher-Rohren, aufgeschweißten Halbrohrprofilen, einem Doppelmantel und/oder einem dem Fachmann bekannten Pillow-Plate-System bzw. Wärmetauscherplatten-System versehen sein, wobei die WärmetauscherRohre sowohl innen- als auch außenliegend ausgeführt sein können.

Stutzen für Zu- und Abläufe können an beliebigen Stellen an Wand, Deckel und Boden des Rührbehälters vorhanden sein. Um das erfindungswesentliche Merkmal zu erfüllen, dass das organische Amin unterhalb des im Reaktionsbehälters vorhandenen Flüssigkeitsspiegels zugegeben wird, ist erfindungsgemäß bevorzugt zumindest eine erste Zuleitung für das organische Amin bzw. die Lösung des organischen Amins derart eingerichtet, dass sie im unteren Bereich in den Reaktor mündet. Dies kann beispielsweise über einen Stutzen im unteren Bereich des Reaktors erreicht werden, der optional mit einer Düse versehen sein kann, durch die das organische Amin bzw. die Lösung des Amins in die Reaktionsmischung eingedüst werden kann. Mittels der vorstehend genannten Zugabe des organischen Amins bzw. der Lösung des Amins, insbesondere durch die Eindüsung, kann beispielsweise bereits eine ausreichende Durchmischung der Suspension erreicht werden. Alternativ oder ergänzend können auch andere Mischvorrichtungen zum Einsatz kommen. Als Mischvorrichtung eignen sich beispielsweise Rührer, Strahldüsen oder Ultraschallmischer. Bevorzugt werden auf einer rotierenden Welle montierte Rührer verwendet. Bei der Verwendung von Strahldüsen ist es beispielsweise denkbar, einen Teil der Reaktionsmischung zu entnehmen und durch die Strahldüse wieder dem Reaktionsbehälter zuzuführen oder aber die Zugabe des Amins bzw. der Aminlösung durch eine solche Strahldüse durchzuführen, so dass es automatisch zu einer Durchmischung der Reaktionsmischung kommt.

In einer bevorzugten Ausführungsform ist ein Verfahren zur Herstellung einer Suspension eines Hydrochlorids eines organischen Amins, umfassend die folgenden Schritte:
(i) Vorlegen mindestens eines organischen Lösungsmittels in einem Reaktionsbehälter unter Bildung eines Flüssigkeitsspiegels,
(ii) Zugeben des organischen Amins,
(iii) Zugeben von Chlorwasserstoff,
wobei die Zugabe des organischen Amins unterhalb des im Reaktionsbehälter vorhandenen Flüssigkeitsspiegels erfolgt und die Schritte (ii) und (iii) zumindest teilweise gleichzeitig durchgeführt werden und die entstehende Suspension während der Aminzugabe durchmischt wird.

Alle weiteren Ausführungsformen in der Beschreibung und den Patentansprüchen können beliebig untereinander und insbesondere mit der vorstehenden Ausführungsform kombiniert werden, sofern sich aus dem Kontext nicht eindeutig das Gegenteil ergibt.

In einer bevorzugten Ausführungsform mündet die Zuleitung über ein Tauchrohr in den Reaktor, so dass bei der Durchführung des erfindungsgemäßen Verfahrens das organische Amin bzw. die Lösung des organischen Amins durch das Tauchrohr direkt in die Reaktionsmischung geleitet wird.

Die vorliegende Erfindung betrifft daher bevorzugt das erfindungsgemäße Verfahren, wobei Schritt (iii) unter Verwendung eines Tauchrohres erfolgt.

Das Verhältnis aus Füllhöhe zum Durchmesser des Rührbehälters liegt erfindungsgemäß bevorzugt bei 0,5:1 bis 1,5:1. Auch höhere Verhältnisse sind möglich, erfordern jedoch in der Regel auch die Verwendung mehrstufiger Mischvorrichtungen, vorzugsweise mehrstufiger Rührorgane. Es ist zu beachten, dass sich durch die Zugabe der Reaktanden im Laufe des Verfahrens die Füllhöhe im Behälter ändert. Besonders bevorzugt, wenn auch nicht zwingend erforderlich, werden die Prozessparameter wie Anfangsfüllstand, Behältergröße und Dosiermengen so gewählt, dass das Verhältnis aus Füllhöhe und Durchmesser des Rührbehälters stets im oben genannten Bereich liegt. Sollte durch die Zugabe des Amins der genannte Bereich verlassen werden, ist es vorteilhaft, eine mehrstufige Mischvorrichtung, vorzugsweise ein mehrstufiges Rührorgan, zu verwenden, so dass die gesamte Reaktionsmischung zu jeder Zeit gut dispergiert wird.

Im Allgemeinen ist es vorteilhaft, wenn die Füllhöhe des vorgelegten Lösungsmittels so hoch ist, dass die Mischvorrichtung, bevorzugt das Rührorgan, in die Flüssigkeit eintaucht, so dass von Beginn an für eine gute Durchmischung und einen möglichst hohen Wärmetransport gesorgt werden kann.

Erfindungsgemäß wird der Chlorwasserstoff bevorzugt als Chlorwasserstoff-Gas eingesetzt. Des Weiteren ist der Reaktor daher bevorzugt mit einer zweiten Zuleitung für Chlorwasserstoff-Gas versehen. Es stehen verschiedene Ausführungsformen für die Zuleitung des Gases zur Verfügung. Falls das Chlorwasserstoff-Gas oben, d.h. oberhalb des Flüssigkeitsspiegels in den Reaktor geleitet wird, so ist es erfindungsgemäß bevorzugt, einen selbstansaugenden Begasungsrührer einzusetzen. Weiter bevorzugt erfolgt die Zugabe über einen Stutzen im unteren Bereich des Reaktors, also unterhalb des Flüssigkeitsspiegels. Besonders bevorzugt ist die Zugabe über ein Einleitrohr, das im Reaktor unterhalb des Rührers, vorzugsweise in einem Gasverteiler mündet, über den das Gas bereits möglichst gut verteilt in die Flüssigkeit unterhalb des Rührers eingetragen wird. Besonders bevorzugt wird auch in diesem Fall ein selbstansaugender Begasungsrührer verwendet, so dass sich eine optimale Dispersion des eingetragenen Chlorwasserstoff-Gases durch die externe Zugabe unterhalb des Rührers und die Verteilung des wieder angesaugten Gases aus dem Gasraum oberhalb der Flüssigkeit ergibt. In einer weiteren bevorzugten Ausführungsform des erfindungsgemäßen Verfahrens wird ein Begasungsrührer mit Hohlwelle für die Einleitung des Chlorwasserstoff-Gases verwendet, wobei die Beschickung der Hohlwelle über die zweite Zuleitung mit, bevorzugt komprimiertem, Gas erfolgt.

Unter selbstansaugenden Begasungsrührern werden erfindungsgemäß solche Rührer verstanden, die über eine Hohlwelle angetrieben werden, welche im oberen Bereich, also oberhalb des Flüssigkeitsspiegels im Reaktor, Öffnungen aufweisen, durch welche Gas aus dem Gasraum in die Hohlwelle angesaugt wird. Das angesaugte Gas wird durch die Hohlwelle nach unten transportiert und tritt durch weitere Öffnungen an den Rührblatt-Organen unterhalb der Flüssigkeitsoberfläche wieder aus, wo es intensiv mit der Flüssigkeit vermischt wird.

Für die Zugabe des organischen Lösungsmittels kann entweder die erste Zuleitung verwendet werden oder alternativ eine weitere, dritte Zuleitung. Weitere Stutzen können zur Prozessüberwachung beispielsweise mittels Sensoren, Schaugläsern oder Probenahme-Vorrichtungen genutzt werden.

Des Weiteren ist der erfindungsgemäß eingesetzte Reaktor bevorzugt mit einem geeigneten Rührer ausgestattet, beispielsweise einem Begasungsrührer, Blattrührer, und/oder einem dynamischen Mischaggregat, ausgewählt aus der Gruppe bestehend aus Dispergierscheiben, Rotor-Stator-Systemen und Kombinationen davon.

Die vorliegende Erfindung betrifft daher bevorzugt das erfindungsgemäße Verfahren, wobei die Schritte (ii) und (iii), unter Rühren mit Hilfe eines Begasungsrührers, eines Blattrührers, eines dynamischen Mischaggregates, ausgewählt aus der Gruppe bestehend aus Dispergierscheiben, Rotor-Stator-Systemen und Kombinationen davon, erfolgt.

Der erfindungsgemäß bevorzugt eingesetzte Reaktor kann des Weiteren auch mindestens eine Abgasleitung aufweisen. Entsprechend geeignete Abgasleitungen sind dem Fachmann an sich bekannt.

Das organische Lösungsmittel, welches erfindungsgemäß in Schritt (i) in den Reaktor gefüllt wird, kann dabei auch bereits gewisse Konzentrationen des umzusetzenden organischen Amins enthalten. Diese Konzentration beträgt beispielsweise 5 Gew.-% oder weniger, bevorzugt 3 Gew.-% oder weniger, besonders bevorzugt 2 Gew.-% oder weniger. Wenn das organische Amin bereits in dem Lösungsmittel gelöst vorliegt, so liegt es bevorzugt in einer Menge von mindestens 0,001 Gew.-% vor. Das organische Amin kann erfindungsgemäß bereits zuvor im Lösungsmittel gelöst sein. Alternativ wird die Lösung erst im Reaktionsbehälter hergestellt.

Besonders bevorzugt ist das mindestens eine organische Lösungsmittel beim Vorlegen in dem Reaktor jedoch bis auf eventuell auftretende Spuren zunächst frei von umzusetzendem, organischem Amin.

In dem erfindungsgemäßen Verfahren wird die Temperatur des organischen Lösungsmittels im Reaktionsbehälter vor Schritt (ii) bevorzugt auf -20 bis 100 °C, besonders bevorzugt 20 bis 90 °C, ganz besonders bevorzugt 30 bis 90 °C, eingestellt.

Schritt (ii) des erfindungsgemäßen Verfahrens umfasst das Zugeben von Chlorwasserstoff.

Chlorwasserstoff (HCl) ist dem Fachmann an sich bekannt und kann erfindungsgemäß als Gas oder in wässriger Lösung als Salzsäure zugegeben werden. Erfindungsgemäß bevorzugt wird Chlorwasserstoff gasförmig zugegeben, gegebenenfalls in Mischung mit einem oder mehreren weiteren Gasen, beispielsweise inerten Gasen wie Stickstoff oder Edelgasen, bevorzugt Stickstoff. In dem Fall, dass Chlorwasserstoff in Mischung mit einem oder mehreren inerten Gasen zugegeben wird, beträgt der Gehalt an Chlorwasserstoff in dem Gasgemisch bevorzugt 5 bis 99 Gew.-%, besonders bevorzugt 50 bis 99 Gew.-%, ganz besonders bevorzugt 80 bis 99 Gew.-%.

Verfahren zur Herstellung von Chlorwasserstoff sind dem Fachmann an sich bekannt, er kann beispielsweise aus den Elementen in einer Chlorknallgasreaktion hergestellt werden oder fällt als Nebenprodukt bei der Chlorierung organischer Verbindungen an.

Chlorwasserstoff wird gasförmig erfindungsgemäß bevorzugt mit einer Temperatur von -20 bis 100 °C, besonders bevorzugt 10 bis 50 °C, zugegeben.

In dem erfindungsgemäß möglichen Fall, dass sich bereits organisches Amin im Reaktionsbehälter befindet, ist es vorteilhaft, zunächst Chlorwasserstoff in einer Menge einzudosieren, die ausreicht, um das vorgelegte organische Amin vollständig in das entsprechende Hydrochlorid zu überführen, bevor mit der Dosierung weiteren organischen Amins gemäß Schritt (iii) begonnen wird.

Es ist erfindungsgemäß auch möglich, mit der Dosierung des Chlorwasserstoffs gleichzeitig mit der Dosierung des organischen Amins (Schritt (iii)) zu beginnen. Aufgrund des höheren Risikos von Agglomerat-Bildungen bei dieser Ausführungsform kann erfindungsgemäß entweder das Chlorwasserstoff-Gas in einem höheren Überschuss bezogen auf das Amin zudosiert werden, oder nach Beendigung Zugabe des organischen Amins wird noch weiter Chlorwasserstoff zuzugeben, bis die gesamte Menge an organischem Amin in das Hydrochlorid überführt wurde. Ein höherer Überschuss bedeutet dabei bevorzugt ein Äquivalentverhältnis von HCl zu Amin von 1,1:1 bis 20, besonders bevorzugt von 1,2:1 bis 5:1.

Bevorzugt wird das Chlorwasserstoffgas unterhalb des Flüssigkeitsspiegels in den Reaktor eingetragen. Besonders bevorzugt erfolgt die Einleitung des Chlorwasserstoffgases unterhalb der Mischvorrichtung, vorzugsweise des Rührorgans, ganz besonders bevorzugt erfolgt die Zugabe durch einen Gasverteiler unterhalb der Mischvorrichtung, vorzugsweise des Rührorgans.

Vorzugsweise ist die Gaseinleitungsvorrichtung so zu positionieren, dass auch bei einer Thrombenbildung die Einleitung stets in die Flüssigkeit erfolgt und nicht in den Gasraum.

Schritt (iii) des erfindungsgemäßen Verfahrens umfasst das Zugeben des organischen Amins.

Als organische Amine können grundsätzlich alle dem Fachmann bekannten Verbindungen mit primären Aminogruppen eingesetzt werden. Erfindungsgemäß bevorzugt sind jedoch solche Verbindungen, die wenigstens 2, besonders bevorzugt 2 oder 3 NH₂-Gruppen aufweisen, die aliphatisch, cycloaliphatisch, araliphatisch oder aromatisch gebunden sein können. Erfindungsgemäß ganz besonders bevorzugt sind solche Amine mit 2 aliphatisch, cycloaliphatisch und/oder araliphatisch gebundenen NH₂-Gruppen.

Geeignete aromatische Amine sind beispielsweise ausgewählt aus der Gruppe bestehend aus den reinen Isomeren oder Isomerengemischen des Diaminotoluols, Diaminodimethylbenzols, Diaminonaphthalins, Diaminobenzols, Diaminodiphenylmethans und Mischungen davon. Besonders bevorzugte aromatische Amine sind ausgewählt aus der Gruppe bestehend aus 2,4-Diaminotoluol, 2,6-Diaminotoluol, 1,5-Diaminonaphthalin, p-Phenylendiamin und Mischungen davon.

Geeignete aliphatische, cycloaliphatische oder araliphatische Amine sind beispielsweise ausgewählt aus der Gruppe bestehend aus 1,4-Diaminobutan, 1,5-Diaminopentan (PDA), 1,6-Diaminohexan (HDA), 1,11-Diaminoundecan, 1-Amino-3,5,5-trimethyl-5-aminomethylcyclohexan (IPDA), Bis(p-aminocyclohexyl)methan (PACM), 1,5-Diamino-2-methylpentan, 2,5-Diamino-2,5-dimethylhexan, 1,4-Diaminocyclohexan, 2,4- Hexahydrotoluylendiamin, 2,6-Hexahydrotoluylendiamin (H6TDA), 1,3-Bis(aminomethyl)benzol (m-XDA), 1,4-Bis(aminomethyl)benzol (p-XDA), Isomeren des Bis(aminomethyl)cyclohexan (H6-XDA), Tetramethylxylylendiamin (TMXDA), Isomeren des Bis(aminomethyl)norbornans (NBDA), Neopentandiamin, 2,4,4-Trimethylhexamethylendiamin, 2,2,4-Trimethylhexamethylendiamin und Mischungen davon.

Die vorliegende Erfindung betrifft daher bevorzugt das erfindungsgemäße Verfahren, wobei das organische Amin ausgewählt ist aus der Gruppe bestehend aus aromatischen Aminen, bevorzugt ausgewählt aus der Gruppe bestehend aus den reinen Isomeren oder Isomerengemischen des Diaminotoluols, Diaminodimethylbenzols, Diaminonaphthalins, Diaminobenzols, Diaminodiphenylmethans und Mischungen davon, aliphatischen, cycloaliphatischen oder araliphatischen Aminen, bevorzugt ausgewählt aus der Gruppe bestehend aus 1,4-Diaminobutan, 1,5-Diaminopentan (PDA), 1,6-Diaminohexan (HDA), 1,11-Diaminoundecan, 1-Amino-3,5,5-trimethyl-5-aminomethylcyclohexan (IPDA), Bis(p-aminocyclohexyl)methan (PACM), 1,5-Diamino-2-methylpentan, 2,5-Diamino-2,5-dimethylhexan, 1,4-Diaminocyclohexan, 2,4-Hexahydrotoluylendiamin, 2,6-Hexahydrotoluylendiamin (H6TDA), 1,3-Bis(aminomethyl)benzol (m-XDA), 1,4-Bis(aminometyhl)benzol (p-XDA), Isomeren des Bis(aminomethyl)cyclohexan (H6-XDA), Tetramethylxylylendiamin (TMXDA), Isomeren des Bis(aminomethyl)norbornans (NBDA), Neopentandiamin, 2,4,4-Trimethylhexamethylendiamin, 2,2,4-Trimethylhexamethylendiamin und Mischungen davon.

Erfindungsgemäß ganz besonders bevorzugte organische Amine sind ausgewählt aus der Gruppe bestehend aus 1,5-Diaminopentan (PDA), 1,6-Diaminohexan (HDA), 1-Amino-3,5,5-trimethyl-5-aminomethylcyclohexan (IPDA), 1,3-Bis(aminomethyl)benzol (m-XDA), Isomeren des Bis(aminomethyl)cyclohexan (H6-XDA), Isomeren des Bis(aminomethyl)norbornans (NBDA), 2,4,4-Trimethylhexamethylendiamin, 2,2,4-Trimethylhexamethylendiamin und Mischungen davon.

In Schritt (iii) des erfindungsgemäßen Verfahrens kann das organische Amin oder die Mischung enthaltend mehr als ein organisches Amin in Substanz oder gelöst in mindestens einem Lösungsmittel zugegeben werden. Als Lösungsmittel sind an sich alle dem Fachmann bekannten Lösungsmittel geeignet, die gegenüber den vorherrschenden Reaktionsbedingungen inert sind. Bevorzugt wird das mindestens eine organische Amin in dem gleichen Lösungsmittel oder Lösungsmittelgemisch gelöst, welches in dem Reaktor vorgelegt wird. Besonders bevorzugte Lösungsmittel, um das mindestens eine organische Amin zu lösen sind daher ausgewählt aus der Gruppe bestehend aus aromatischen Kohlenwasserstoffen, halogenierten aromatischen Kohlenwasserstoffen, insbesondere chlorierten aromatischen Kohlenwasserstoffen, Estern, Ethern und halogenierten Kohlenwasserstoffen und Mischungen davon. Erfindungsgemäß besonders bevorzugt eingesetzte aromatische Kohlenwasserstoffe sind ausgewählt aus der Gruppe bestehend aus Toluol, Brombenzol, Chlorbenzol, Dichlorbenzol, beispielsweise ortho-Dichlorbenzol, und Mischungen davon. Besonders bevorzugt werden erfindungsgemäß Chlorbenzol, Dichlorbenzol oder Mischungen davon eingesetzt.

Gemäß dem vorliegenden Verfahren wird das organische Amin bevorzugt gelöst in mindestens einem Lösungsmittel, bevorzugt ausgewählt aus der Gruppe bestehend aus aromatischen Kohlenwasserstoffen, halogenierten aromatischen Kohlenwasserstoffen, insbesondere chlorierten aromatischen Kohlenwasserstoffen, Estern, Ethern und halogenierten Kohlenwasserstoffen und Mischungen davon, insbesondere ausgewählt aus der Gruppe bestehend aus Toluol, Brombenzol, Chlorbenzol, Dichlorbenzol, beispielsweise ortho-Dichlorbenzol, und Mischungen davon, eingesetzt.

Bevorzugt wird das organische Amin als Lösung in einem inerten Lösungsmittel zugegeben. Die Konzentration des organischen Amins in dem Lösungsmittel beträgt bevorzugt 5 bis 50 Gew.-%, besonders bevorzugt 10 bis 30 Gew.-%, jeweils bezogen auf die Lösung. Auf diese Weise kann erfindungsgemäß die Bildung von Agglomeraten aufgrund von lokal überhöhten Konzentrationen des organischen Amins weiter minimiert werden.

Ein erfindungswesentliches Merkmal ist, dass die Zugabe des organischen Amins unterhalb des im Reaktionsbehälter vorhandenen Flüssigkeitsspiegels erfolgt, wobei der Eintrag vorzugsweise über ein Tauchrohr erfolgt. Erfindungsgemäß bedeutet unterhalb des im Reaktionsbehälter vorhandenen Flüssigkeitsspiegels vorzugsweise, dass die Einleitung durchgehend in die im Reaktionsbehälter befindliche Reaktionsmischung erfolgt.

Ein weiteres erfindungswesentliches Merkmal ist, dass die Schritte (ii) und (iii) zumindest teilweise, bevorzugt vollständig, gleichzeitig durchgeführt werden. Zumindest teilweise bedeutet im Rahmen der vorliegenden Erfindung, dass die Zugabe bevorzugt zu 40 bis 100%, besonders bevorzugt 80 bis 99%, besonders bevorzugt 80 bis 95% gleichzeitig erfolgt, d.h. dass dieser Massenanteil des gesamten zuzugebenden organischen Amins in den Reaktor geleitet wird während auch Chlorwasserstoff eingeleitet wird, um mit dem organischen Amin zu dem entsprechenden Hydrochlorid zu reagieren. Es ist erfindungsgemäß aber auch möglich, dass organisches Amin oder Chlorwasserstoff teilweise vorgelegt werden und dann die gleichzeitige Zugabe erfolgt.

Die Dosierung des organischen Amins und des Chlorwasserstoffs erfolgt in einer bevorzugten Ausführungsform des erfindungsgemäßen Verfahrens derart, dass der Chlorwasserstoff im stöchiometrischen Überschuss bezogen auf die dosierten Aminogruppen des organischen Amins zugegeben wird. Auf diese Weise wird verhindert, dass sich das organische Amin zu unerwünscht hohen Konzentrationen anreichert, und es wird eine feinteilige Suspension erhalten.

Während beide Reaktanden dosiert werden, stehen die Stoffmengenströme von Chlorwasserstoff und organischem Amin bevorzugt in einem Äquivalentverhältnis von 1:1 bis 10:1, besonders bevorzugt 1:1 bis 3:1, ganz besonders bevorzugt 1,05:1 bis 1,8:1, zueinander.

Bevorzugt ist die insgesamt dosierte Menge an Chlorwasserstoff ausreichend, um das organische Amin vollständig, d.h. zu mehr als 99%, vorzugsweise mehr als 99,5% und besonders bevorzugt mehr als 99,9% in das Hydrochlorid zu überführen.

Die Endkonzentration an Hydrochlorid in der Reaktionsmischung liegt bevorzugt bei 5 is 30 Gew.-%, besonders bevorzugt 7 bis 25 Gew.-%, ganz besonders bevorzugt 10 bis 20 Gew.-%. Der Wert für die Endkonzentration an Hydrochlorid ergibt sich rechnerisch aus der Masse des im Reaktor vorhandenen organischen Amins unter Berücksichtigung des Molekulargewichtsanstiegs durch die Bildung des Hydrochlorids, wobei eine vollständige Umsetzung aller Aminogruppen in die entsprechenden Ammoniumchlorid-Gruppen angenommen wird, bezogen auf die Gesamtmasse des Reaktorinhalts. Bei den genannten erfindungsgemäß bevorzugten Konzentrationsbereichen liegt ein besonders vorteilhafter Kompromiss von hoher Wirtschaftlichkeit des Verfahrens und guter Handhabbarkeit der Suspensionen vor.

Nach der erfindungsgemäßen Herstellung des Hydrochlorids umfassend die Schritte (i), (ii) und (iii) wird die nach Schritt (iii) erhaltene Suspension bevorzugt in einem Schritt (iv) mit Phosgen unter Erhalt des dem eingesetzten organischen Amins entsprechenden organischen Isocyanates umgesetzt.

Die vorliegende Erfindung betrifft daher bevorzugt das erfindungsgemäße Verfahren, wobei die nach Schritt (iii) erhaltene Suspension in einem Schritt iv) mit Phosgen unter Erhalt des dem eingesetzten organischen Amins entsprechenden organischen Isocyanates umgesetzt wird.

Die nach dem erfindungsgemäßen Verfahren erhaltene Suspension des Hydrochlorids kann anschließend durch Reaktion mit Phosgen zu dem entsprechenden Isocyanat umgesetzt werden. Hierzu kann die Suspension entweder im Reaktionsbehälter verbleiben oder in einen weiteren Reaktionsbehälter überführt werden.

Für den Phosgenierungsschritt können die gleichen Arten von Reaktionsbehältern verwendet werden wie zuvor für die Herstellung der Hydrochlorid-Suspension beschrieben. Die Suspension wird dann unter Einleitung von Phosgen, gegebenenfalls verdünnt mit einem inerten Gas oder Gasgemisch, beispielsweise Stickstoff oder Edelgase, auf eine Temperatur von 120 bis 200 °C erhitzt. Insbesondere für thermisch weniger stabile Isocyanate ist es bevorzugt, dass diese Temperatur 120 bis 170 °C, besonders bevorzugt 120 bis 160 °C beträgt.

Erfindungsgemäß bevorzugt erfolgt deshalb die Phosgenierung bei einer Temperatur von 120 bis 170 °C, besonders bevorzugt 130 bis 160 °C. Die Phosgenierung kann sowohl bei Atmosphärendruck als auch bei Überdruck erfolgen. Vorzugsweise liegt ein Druck bei 1 bis 10 bar(a), besonders bevorzugt 1 bis 5 bar(a).

Phosgen wird für die Phosgenierung bevorzugt im stöchiometrischen Überschuss eingesetzt. Höhere Überschüsse wirken sich positiv auf die Reaktionsgeschwindigkeit und damit die Dauer der Umsetzung aus, beeinträchtigen jedoch naturgemäß die Wirtschaftlichkeit des Verfahrens. Üblicherweise wird die Reaktion deshalb mit einem Phosgenüberschuss von 100 bis 500%, bevorzugt 150 bis 300%, durchgeführt. Optional kann auch ein Inertgas in die Reaktionsmischung eingeleitet werden.

Die Zugabe des Phosgens erfolgt vorzugsweise durch Einleiten von gasförmigem Phosgen in die Hydrochlorid-Suspension. Bevorzugt wird das Phosgen unterhalb des Flüssigkeitsspiegels in den Reaktor eingetragen. Besonders bevorzugt erfolgt die Einleitung des Phosgens unterhalb der Mischvorrichtung, ganz besonders bevorzugt erfolgt die Zugabe durch einen Gasverteiler unterhalb der Mischvorrichtung. Als Mischvorrichtung werden bevorzugt Begasungsrührer mit Hohlwelle verwendet, wie zuvor bereits beschrieben.

In einer alternativen Ausführungsform wird das Phosgen als Lösung in einem inerten Lösungsmittel in den Reaktor eingebracht. Bevorzugt wird hierzu das gleiche Lösungsmittel verwendet, in dem auch die Suspension des Hydrochlorids vorliegt.

In einer weiteren alternativen Ausführungsform ist es auch möglich, eine Phosgen-Lösung in einem Reaktionsbehälter vorzulegen und die erfindungsgemäß hergestellte Hydrochlorid-Suspension zu dieser Lösung zu geben.

Es kann vorteilhaft sein, die Zuleitungen für Amin und/oder Chlorwasserstoff vor und/oder nach der Reaktion mit Lösungsmittel und/oder Inertgas, bevorzugt Stickstoff, zu spülen.

Nach Beendigung der Reaktion, d.h. bei weitgehend vollständigem Umsatz, d.h. bevorzugt 80 bis 99%, besonders bevorzugt 90 bis 99%, des theoretischen Wertes, des Hydrochlorids zum entsprechenden Isocyanat, werden bevorzugt überschüssiges Phosgen und Chlorwasserstoff aus der Reaktionsmischung entfernt. Hierzu wird in der Regel ein Inertgas, bevorzugt Stickstoff, durch die Reaktionsmischung geleitet. Optional kann das Entfernen von Phosgen und Chlorwasserstoff auch durch Anlegen eines Unterdrucks erfolgen oder unterstützt werden. Bei Bedarf kann eine Filtration erfolgen, um gegebenenfalls vorhandene Feststoffe wie beispielsweise nicht umgesetzte Hydrochlorid-Partikel zu entfernen.

Anschließend wird das Reaktionsprodukt bevorzugt gemäß aus dem Stand der Technik bekannten Verfahren aufgearbeitet, d.h. es wird bevorzugt in einer mehrstufigen Vakuumdestillation gereinigt. Insbesondere wird bei dieser destillativen Aufarbeitung das Isocyanat von Lösungsmittel, chlorierten Nebenprodukten und höhersiedenden Rückständen befreit.

Da zur Herstellung der Isocyanate bevorzugt Lösungsmittel verwendet werden, die einen niedrigeren Siedepunkt als das jeweilige Isocyanat aufweisen, umfasst die Destillation bevorzugt eine Lösungsmittelabtrennung. In diesem Destillationsschritt können auch leichtsiedende Nebenkomponenten, insbesondere chlorierte leichtsiedende Nebenkomponenten, abgetrennt werden. Das Lösungsmittel kann, gegebenenfalls nach weiteren Reinigungsschritten, wieder in den Prozess zurückgeführt werden.

Außerdem umfasst ein solches Reinigungsverfahren bevorzugt eine Reindestillation zur Abtrennung des Isocyanates von schwersiedendem Rückstand. Alle Destillationsschritte erfolgen bevorzugt im Vakuum, um die erforderlichen Temperaturen für die Destillation und damit die thermische Belastung des Produkts zu verringern. Insbesondere wird dieser Destillationsschritt bei einem Druck von 5 bis 50 mbar(a) und einer Sumpf-Temperatur von 90 bis 250 °C, bevorzugt 120 bis 170 °C, durchgeführt. Um bei Bedarf die Bildung von Uretdionen im Destillat zurückzudrängen, erfolgt die Abkühlung des Destillates bevorzugt möglichst rasch auf Temperaturen unterhalb von 90 °C, vorzugsweise unterhalb von 80 °C. Dies kann beispielsweise erreicht werden, indem das Destillat abgeschreckt wird, insbesondere durch Vermischen mit bereits gekühltem Produkt.

Nach Schritt (iv) des erfindungsgemäßen Verfahrens wird bevorzugt das organische Isocyanat, insbesondere 1,5-Diisocyanatopentan (PDI), 1,6-Diisocyanatohexan (HDI), 1-Isocyanato-3,5,5-trimethyl-5-isocyanatomethylcyclohexan (IPDI), 1,3-Bis(isocyanatomethyl)benzol (m-XDI), Isomeren des Bis(isocyanatomethyl)cyclohexan (H6-XDI), Isomeren des Bis(isocyanatomethyl)norbornans (NBDI), 2,4,4-Trimethylhexamethylendiamin, 2,2,4-Trimethylhexamethylendiamin oder Mischungen davon, in reiner Form, d.h. in einer Reinheit von mindestens 99,5%, erhalten.

Auch wenn nicht Teil der Erfindung wird ein Isocyanat beschrieben, erhältlich nach dem erfindungsgemäßen Verfahren. Insbesondere wird ein organisches Isocyanat beschrieben, ausgewählt aus der Gruppe bestehend aus 1,5-Diisocyanatopentan (PDI), 1,6-Diisocyanatohexan (HDI), 1-Isocyanato-3,5,5-trimethyl-5-isocyanatomethylcyclohexan (IPDI), 1,3-Bis(isocyanatomethyl)benzol (m-XDI), Isomeren des Bis(isocyanatomethyl)cyclohexan (H6-XDI), Isomeren des Bis(Isocyanatomethyl)norbornans (NBDI), 2,4,4-Trimethylhexamethylendiamin, 2,2,4-Trimethylhexamethylendiamin und Mischungen davon organisches Isocyanat, erhältlich nach dem erfindungsgemäßen Verfahren.

Ebenfalls beschrieben wird die Verwendung des organischen Isocyanates zur Herstellung von Isocyanuratgruppen aufweisenden Polyisocyanaten, Uretdiongruppen aufweisenden Polyisocyanaten, Biuretgruppen aufweisenden Polyisocyanaten, Urethan- oder Allophanatgruppen aufweisenden Polyisocyanaten, Oxadiazintriongruppen oder Iminooxadiazindiongruppen enthaltenden Polyisocyanaten und/oder Uretonimin-modifizierten Polyisocyanaten.

Derartige Polyisocyanate auf Basis des organischen Isocyanates oder das organische Isocyanat selbst können beispielsweise zur Herstellung von Urethan-, Thiourethan-, Isocyanurat-, Amid- und/oder Harnstoffgruppen enthaltenden Kunststoffen nach dem Polyisocyanat-Polyadditionsverfahren verwendet werden. Derartige Polyisocyanatmischungen werden insbesondere zur Herstellung von lichtbeständigen Polyurethanlacken und -überzügen verwendet.

### Beispiele:

### Vergleichsbeispiel 1

Ein Rührbehälter aus Glas mit einem Temperiermantel und einem Länge-zu-Innendurchmesser-Verhältnis von 1,2 wurde mit einem Blattrüher ausgerüstet, dessen Rührblatt-Durchmesser zum Innendurchmesser des Reaktors in einem Verhältnis von 0,75 stand. Der Rührbehälter wurde mit 5 kg Monochlorbenzol beschickt. Gleichzeitig wurde in einer Rohstoffvorlage 1 kg m-Xylylendiamin in 4 kg o-Dichlorbenzol vorgelegt. Unter Rühren wurden dann bei Atmosphärendruck gleichzeitig Chlorwasserstoffgas über ein Gas-Einleitungs-Rohr in den Reaktor geleitet, und die in der Rohstoffvorlage bereitgestellte Amin-Lösung zugegeben, wobei die Zugabe der Amin-Lösung von oben, also oberhalb des Flüssigkeitsspiegels in den Rührbehälter erfolgte. Die Dosierung erfolgte über 2 h mit einer Dosierrate von 321 g/h für Chlorwasserstoff und 2,5 kg/h für die Amin-Lösung. Dann wurde die Amin-Zufuhr beendet und noch weitere 60 min Chlorwasserstoff mit einer Dosierrate von 150 g/h zugeführt. Die Temperatur im Rührbehälter wurde während des ganzen Vorgangs über den Temperiermantel auf 25 °C gehalten.

Es zeigten sich starke, klebrige Ablagerungen an der Wandung des Rührbehälters, am Rührer und am Einleitstutzen der Aminlösung. Die erhaltene Reaktionsmischung enthielt große Feststoff-Agglomerate mit Durchmessern > 1 mm, die sich absetzten. Die erhaltene Reaktionsmischung war daher nicht mehr rühr- und pumpfähig.

### Beispiel 2 (erfindungsgemäß)

Der Rührbehälter gemäß Vergleichsbeispiel 1 wurde modifziert, so dass nun der Zulauf der Amin-Lösung durch ein Tauchrohr direkt in die Reaktionsmischung und somit die Zugabe unterhalb des Flüssigkeitsspiegels erfolgte. Außerdem wurde nach Beendigung der Amin-Zugabe nur noch für 30 Minuten Chlorwasserstoff mit einer Dosierrate von 150 g/h zugeführt. Der Versuch aus Vergleichsbeispiel 1 wurde unter ansonsten unveränderten Bedingungen wiederholt. Die Temperatur im Rührbehälter wurde über den Temperiermantel auf 25 °C gehalten.

Es zeigten sich nur noch geringe Ablagerungen an der Wandung des Rührbehälters. Der Rührer und das Tauchrohr zur Einleitung waren frei von Verklebungen. Die erhaltene Suspension war rühr- und pumpfähig, enthielt jedoch noch größere Feststoffpartikel deren Größe jedoch unterhalb von 1 mm Durchmesser lag.

### Beispiel 3 (erfindungsgemäß)

Für einen weiteren Versuch wurde der Rührbehälter erneut modifziert, indem der Blattrührer durch einen Begasungsrührer ersetzt wurde. Der Versuch aus Beispiel 2 (erfindungsgemäß) wurde wiederholt, wobei nun die Einleitung des Chlorwasserstoffs durch den Begasungsrührer erfolgte. Außerdem wurde der Reaktor über den Temperiermantel auf 35 °C vorgewärmt und während der Zugabe der Reaktanden auf dieser Temperatur gehalten. Nach Beendigung des Versuchs wurden wiederum nur geringe Ablagerungen an der Wandung des Rührbehälters beobachtet, während Rührer und Tauchrohr frei von Ablagerungen waren. Die erhaltene Suspension war homogen feinteilig sowie rühr- und pumpfähig.

### Beispiel 4 (erfindungsgemäß)

Der Versuch aus Beispiel 3 (erfindungsgemäß) wurde wiederholt, wobei abweichend von den Bedingungen in Beispiel 3 der Reaktor über den Temperiermantel auf 80 °C vorgewärmt und während der Zugabe der Reaktanden auf dieser Temperatur gehalten wurde. Es ergab sich wiederum eine homogen feinteilige sowie rühr- und pumpfähige Suspension und auch Ablagerungen an der Wandung des Reaktors konnten weiter minimiert werden.

### Beispiel 5 (erfindungsgemäß)

Es wurde verfahren wie in Beispiel 4, jedoch wurde als Amin ein Isomerengemisch aus 2,2,4- und 2,4,4-Trimethylhexamethylendiamin eingesetzt. Dieses wurde in der Rohstoffvorlage als 20 Gew.-%ige Lösung in Monochlorbenzol angesetzt. Die Temperatur wurde wiederum auf 80 °C geregelt und die Dosierraten betrugen 2,5 kg/h für die Amin-Lösung und 275 g/h für Chlorwasserstoff. Nach 2 Stunden wurde die Dosierung des Amins beendet und es wurde noch für weitere 30 min Chlorwasserstoff mit einer Dosierrate von 100 g/h zugeführt. Es wurde erneut eine homogen feinteilige sowie rühr- und pumpfähige Suspension erhalten. Ablagerungen traten nicht auf.

### Vergleichsbeispiel 6

Abweichend von Beispiel 5 wurde in Vergleichsbeispiel 6 der Reaktor zunächst mit 10 kg einer 10%igen Lösung des Isomerengemisches aus 2,2,4- und 2,4,4-Trimethylhexamethylendiamin in Monochlorbenzol beschickt und auf 25 °C temperiert. Dann wurde unter intensivem Rühren 2,5 h lang Chlorwasserstoff-Gas mit einer Dosierrate von 275 g/h eingeleitet. Es kam zu starken Ablagerungen an Wandungen und Rührern sowie zur Bildung größerer Feststoff-Agglomerate mit Durchmessern > 1 mm, die sich am Boden des Behälters absetzten.

### Beispiel 7 (erfindungsgemäß)

In der Rohstoffvorlage wurden 900 g des Isomerengemisches aus 2,2,4- und 2,4,4-Trimethylhexamethylendiamin in 4 kg Monochlorbenzol vorgelegt. Im Rührbehälter wurden weitere 100 g des Amins in 5 kg Monochlorbenzol vorgelegt. Die Reaktionsmischung wurde auf 60 °C temperiert und unter Rühren zunächst durch den Begasungsrührer mit 70 g Chlorwasserstoff versetzt. Erst dann wurde mit der gleichzeitigen Dosierung der Aminlösung und weiteren Chlorwasserstoffs begonnen. Die Dosierraten betrugen 2,45 kg/h für die Amin-Lösung, welche erfindungsgemäß durch ein Tauchrohr unterhalb des Flüssigkeitsspiegels zugeführt wurde und 250 g/h für den Chlorwasserstoff. Nach 2 h wurde die Amin-Zugabe beendet und Chlorwasserstoff für weitere 30 min mit einer reduzierten Dosierrate von 100 g/h zudosiert. Nach Beendigung des Versuchs wurden wiederum nur geringe Ablagerungen an der Wandung des Rührbehälters beobachtet, während Rührer und Tauchrohr frei von Ablagerungen waren. Die erhaltene Suspension war homogen feinteilig sowie rühr- und pumpfähig.

### Beispiel 8 (erfindungsgemäß)

In einem wie in Beispiel 3 beschrieben modifizierten Rührbehälter wurden 5000 g Monochlorbenzol vorgelegt und der Reaktor wurde über den Temperiermantel auf 70 °C vorgewärmt und während der Zugabe der Reaktanden auf dieser Temperatur gehalten. Als Aminlösung wurde aus der Rohstoffvorlage über das Tauchrohr eine Lösung von 1000 g Anilin in 4000 g Monochlorbenzol zugegeben. Chlorwasserstoffgas wurde durch den Begasungsrührer eingeleitet. Die Dosierraten betrugen 2 kg/h für die Amin-Lösung und 500 g/h für Chlorwasserstoff. Nach 2,5 h war alles Amin zugegeben und es wurde noch für 30 Minuten mit einer Dosierrate von 100 g/h weiter Chlorwasserstoff eingeleitet. Es wurde eine homogen feinteilige sowie rühr- und pumpfähige Suspension erhalten. Ablagerungen traten nicht auf.

### Beispiel 9 (erfindungsgemäß)

Der Versuch aus Beispiel 5 wurde wiederholt, wobei statt o-Dichlorbenzol in diesem Fall Monochlorbenzol vorgelegt wurde und die Amin-Lösung in o-Dichlorbenzol angesetzt wurde. Die weiteren Bedingungen blieben unverändert. Es wurde erneut eine homogen feinteilige sowie rühr- und pumpfähige Suspension erhalten. Ablagerungen traten nicht auf.

## Patentansprüche

1. Verfahren zur Herstellung einer Suspension eines Hydrochlorids eines organischen Amins, umfassend die folgenden Schritte:
(i) Vorlegen mindestens eines organischen Lösungsmittels in einem Reaktionsbehälter unter Bildung eines Flüssigkeitsspiegels,
(ii) Zugeben von Chlorwasserstoff,
(iii) Zugeben des organischen Amins,
**dadurch gekennzeichnet, dass** die Zugabe des organischen Amins unterhalb des im Reaktionsbehälters vorhandenen Flüssigkeitsspiegels erfolgt und die Schritte (ii) und (iii) zumindest teilweise gleichzeitig durchgeführt werden.

2. Verfahren gemäß Anspruch 1, **dadurch gekennzeichnet, dass** die Temperatur des organischen Lösungsmittels im Reaktionsbehälter vor Schritt (ii) auf -20 bis 100 °C, bevorzugt 20 bis 90 °C, besonders bevorzugt 30 bis 90 °C, eingestellt wird.

3. Verfahren gemäß Anspruch 1 oder 2, **dadurch gekennzeichnet, dass** Schritt (iii) unter Verwendung eines Tauchrohres erfolgt.

4. Verfahren nach einem der Ansprüche 1 bis 3, **dadurch gekennzeichnet, dass** das mindestens eine organische Lösungsmittel ausgewählt ist aus der Gruppe bestehend aus aromatischen Kohlenwasserstoffen, halogenierten aromatischen Kohlenwasserstoffen, insbesondere chlorierten aromatischen Kohlenwasserstoffen, Estern, Ethern, halogenierten Kohlenwasserstoffen und Mischungen davon.

5. Verfahren nach einem der Ansprüche 1 bis 4, **dadurch gekennzeichnet, dass** das organische Amin als Lösung in einem inerten Lösungsmittel zugegeben wird.

6. Verfahren nach Anspruch 5, **dadurch gekennzeichnet, dass** die Konzentration des organischen Amins in dem Lösungsmittel 5 bis 50 Gew.-%, bevorzugt 10 bis 30 Gew.-%, jeweils bezogen auf die Lösung, beträgt.

7. Verfahren nach einem der Ansprüche 1 bis 6, **dadurch gekennzeichnet, dass** die Stoffmengenströme von Chlorwasserstoff und organischem Amin während der Schritte (ii) und (iii) in einem Äquivalentverhältnis von 1:1 bis 10:1, bevorzugt 1:1 bis 3:1, besonders bevorzugt 1,05:1 bis 1,8:1, zueinander stehen.

8. Verfahren nach einem der Ansprüche 1 bis 7, **dadurch gekennzeichnet, dass** die Endkonzentration an Hydrochlorid in der Reaktionsmischung bei 5 is 30 Gew.-%, bevorzugt 7 bis 25 Gew.-%, besonders bevorzugt 10 bis 20 Gew.-%, liegt.

9. Verfahren gemäß einem der Ansprüche 1 bis 8, **dadurch gekennzeichnet, dass** die Schritte (ii) und (iii) unter Rühren mit Hilfe eines Begasungsrührers, Blattrührers oder eines dynamischen Mischaggregates ausgewählt aus der Gruppe bestehend aus Dispergierscheiben, Rotor-Stator-Systemen und Kombinationen davon, erfolgt.

10. Verfahren nach einem der Ansprüche 1 bis 9, **dadurch gekennzeichnet, dass** das organische Amin ausgewählt ist aus der Gruppe bestehend aus aromatischen Aminen, bevorzugt ausgewählt aus der Gruppe bestehend aus den reinen Isomeren oder Isomerengemischen des Diaminotoluols, Diaminodimethylbenzols, Diaminonaphthalins, Diaminobenzols, Diaminodiphenylmethans und Mischungen davon, aliphatischen, cycloaliphatischen oder araliphatischen Aminen, bevorzugt ausgewählt aus der Gruppe bestehend aus 1,4-Diaminobutan, 1,5-Diaminopentan (PDA), 1,6-Diaminohexan (HDA), 1,11-Diaminoundecan, 1-Amino-3,5,5-trimethyl-5-aminomethylcyclohexan (IPDA), Bis(p-aminocyclohexyl)methan (PACM), 1,5-Diamino-2-methylpentan, 2,5-Diamino-2,5-dimethylhexan, 1,4-Diaminocyclohexan, 2,4- Hexahydrotoluylendiamin, 2,6-Hexahydrotoluylendiamin (H6TDA), 1,3-Bis(aminomethyl)benzol (m-XDA), 1,4-Bis(aminometyhl)benzol (p-XDA), Isomeren des Bis(aminomethyl)cyclohexan (H6-XDA), Tetramethylxylylendiamin (TMXDA), Isomeren des Bis(aminomethyl)norbornans (NBDA), Neopentandiamin, 2,4,4-Trimethylhexamethylendiamin, 2,2,4-Trimethylhexamethylendiamin und Mischungen davon.

11. Verfahren nach einem der Ansprüche 1 bis 10, **dadurch gekennzeichnet, dass** die nach Schritt (iii) erhaltene Suspension in einem Schritt (iv) mit Phosgen unter Erhalt des dem eingesetzten organischen Amins entsprechenden organischen Isocyanates umgesetzt wird.

## Claims

1. Method for preparing a suspension of a hydrochloride of an organic amine, comprising the following steps:
(i) initially charging at least one organic solvent in a reaction vessel to form a liquid level,
(ii) adding hydrogen chloride,
(iii) adding the organic amine,
**characterized in that** the organic amine is added below the liquid level present in the reaction vessel and steps (ii) and (iii) are at least partly carried out simultaneously.

2. Method according to Claim 1, **characterized in that** the temperature of the organic solvent in the reaction vessel prior to step (ii) is adjusted to - 20 to 100°C, preferably 20 to 90°C, particularly preferably 30 to 90°C.

3. Method according to Claim 1 or 2, **characterized in that** step (iii) is carried out using an immersion tube.

4. Method according to any of Claims 1 to 3, **characterized in that** the at least one organic solvent is selected from the group consisting of aromatic hydrocarbons, halogenated aromatic hydrocarbons, particularly chlorinated aromatic hydrocarbons, esters, ethers, halogenated hydrocarbons and mixtures thereof.

5. Method according to any of Claims 1 to 4, **characterized in that** the organic amine is added as a solution in an inert solvent.

6. Method according to Claim 5, **characterized in that** the concentration of the organic amine in the solvent is 5 to 50% by weight, preferably 10 to 30% by weight, based in each case on the solution.

7. Method according to any of Claims 1 to 6, **characterized in that** the substance streams of hydrogen chloride and organic amine during steps (ii) and (iii) are in an equivalence ratio to each other of from 1:1 to 10:1, preferably 1:1 to 3:1, particularly preferably 1.05:1 to 1.8:1.

8. Method according to any of Claims 1 to 7, **characterized in that** the end concentration of hydrochloride in the reaction mixture is 5 to 30% by weight, preferably 7 to 25% by weight, particularly preferably 10 to 20% by weight.

9. Method according to any of Claims 1 to 8, **characterized in that** steps (ii) and (iii) are carried out while stirring with the aid of a sparging stirrer, paddle stirrer or a dynamic mixer selected from the group consisting of disperser disks, rotor-stator systems and combinations thereof.

10. Method according to any of Claims 1 to 9, **characterized in that** the organic amine is selected from the group consisting of aromatic amines, preferably selected from the group consisting of pure isomers or isomeric mixtures of diaminotoluene, diaminodimethylbenzene, diaminonaphthalene, diaminobenzene, diaminodiphenylmethane and mixtures thereof, aliphatic, cycloaliphatic or araliphatic amines, preferably selected from the group consisting of 1,4-diaminobutane, 1,5-diaminopentane (PDA), 1,6-diaminohexane (HDA), 1,11-diaminoundecane, 1-amino-3,5,5-trimethyl-5-aminomethylcyclohexane (IPDA), bis(p-aminocyclohexyl)methane (PACM), 1,5-diamino-2-methylpentane, 2,5-diamino-2,5-dimethylhexane, 1,4-diaminocyclohexane, 2,4-hexahydrotoluylenediamine, 2,6-hexahydrotoluylenediamine (H6TDA), 1,3-bis(aminomethyl)benzene (m-XDA), 1,4-bis(aminomethyl)benzene (p-XDA), isomers of bis(aminomethyl)cyclohexane (H6-XDA), tetramethylxylylenediamine (TMXDA), isomers of bis(aminomethyl)norbornane (NBDA), neopentanediamine, 2,4,4-trimethylhexamethylenediamine, 2,2,4-trimethylhexamethylenediamine and mixtures thereof.

11. Method according to any of Claims 1 to 10, **characterized in that** the suspension obtained after step (iii) is reacted in a step (iv) with phosgene to obtain the organic isocyanate corresponding to the organic amine used.

## Revendications

1. Procédé pour la production d'une suspension d'un chlorhydrate d'une amine organique, comprenant les étapes suivantes :
(i) disposition au préalable d'au moins un solvant organique dans un récipient de réaction avec formation d'un niveau de liquide,
(ii) addition de chlorure d'hydrogène,
(iii) addition de l'amine organique,
**caractérisé en ce que** l'addition de l'amine organique s'effectue au-dessous du niveau de liquide présent dans le récipient de réaction et les étapes (ii) et (iii) sont effectuées au moins en partie simultanément.

2. Procédé selon la revendication 1, **caractérisé en ce que** la température du solvant organique dans le récipient de réaction avant l'étape (ii) est ajustée à -20 à 100 °C, de préférence 20 à 90 °C, de façon particulièrement préférée 30 à 90 °C.

3. Procédé selon la revendication 1 ou 2, **caractérisé en ce que** l'étape (iii) s'effectue avec utilisation d'un tube plongeur.

4. Procédé selon l'une quelconque des revendications 1 à 3, **caractérisé en ce que** ledit au moins un solvant organique est choisi dans le groupe constitué par les hydrocarbures aromatiques, les hydrocarbures aromatiques halogénés, en particulier les hydrocarbures aromatiques chlorés, les esters, les éthers, les hydrocarbures halogénés et des mélanges de ceux-ci.

5. Procédé selon l'une quelconque des revendications 1 à 4, **caractérisé en ce que** l'amine organique est ajoutée sous forme de solution dans un solvant inerte.

6. Procédé selon la revendication 5, **caractérisé en ce que** la concentration de l'amine organique dans le solvant vaut de 5 à 50 % en poids, de préférence 10 à 30 % en poids, chaque fois par rapport à la solution.

7. Procédé selon l'une quelconque des revendications 1 à 6, **caractérisé en ce que** les flux de quantités de substances de chlorure d'hydrogène et d'amine organique pendant les étapes (ii) et (iii) se situent l'un par rapport à l'autre en un rapport d'équivalents de 1:1 à 10:1, de préférence 1:1 à 3:1, de façon particulièrement préférée 1,05:1 à 1,8:1.

8. Procédé selon l'une quelconque des revendications 1 à 7, **caractérisé en ce que** la concentration finale de chlorhydrate dans le mélange réactionnel vaut de 5 à 30 % en poids, de préférence 7 à 25 % en poids, de façon particulièrement préférée 10 à 20 % en poids.

9. Procédé selon l'une quelconque des revendications 1 à 8, **caractérisé en ce que** les étapes (ii) et (iii) s'effectuent sous agitation à l'aide d'un agitateur de gazage, d'un agitateur à palette ou d'un appareil de mélange dynamique choisi dans le groupe constitué par les disques de dispersion, les systèmes à rotor-stator et des combinaisons de ceux-ci.

10. Procédé selon l'une quelconque des revendications 1 à 9, **caractérisé en ce que** l'amine organique est choisie dans le groupe constitué par les amines aromatiques, de préférence choisies dans le groupe constitué par les isomères purs ou mélanges d'isomères du diaminotoluène, diaminodiméthylbenzène, diamino-naphtalène, diaminobenzène, diaminodiphénylméthane et des mélanges de ceux-ci, les amines aliphatiques, cycloaliphatiques ou araliphatiques, de préférence choisies dans le groupe constitué par le 1,4-diaminobutane, le 1,5-diaminopentane (PDA), le 1,6-diaminohexane (HDA), le 1,11-diamino-undécane, le 1-amino-3,5,5-triméthyl-5-aminométhylcyclohexane (IPDA), le bis(p-aminocyclohexyl)méthane (PACM), le 1,5-diamino-2-méthylpentane, le 2,5-diamino-2,5-dimethyl-hexane, le 1,4-diaminocyclohexane, la 2,4-hexahydrotoluylènediamine, la 2,6-hexahydrotoluylènediamine (H6TDA), le 1,3-bis(aminométhyl)benzène (m-XDA), le 1,4-bis(aminométhyl)benzène (p-XDA), les isomères du bis(aminométhyl)cyclohexane (H6-XDA), la tétraméthylxylylènediamine (TMXDA), les isomères du bis(aminométhyl)norbornane (NBDA), la néopentanediamine, la 2,4,4-triméthylhexaméthylènediamine, la 2,2,4-triméthylhexaméthylènediamine et des mélanges de ceux-ci.

11. Procédé selon l'une quelconque des revendications 1 à 10, **caractérisé en ce que** dans une étape (iv) on fait réagir la suspension obtenue après l'étape (iii) avec du phosgène, pour obtenir l'isocyanate organique correspondant à l'amine organique utilisée.
